# EUROPEAN PATENT SPECIFICATION

(11) **EP 0 567 508 B1**
(45) Date of publication and mention of the grant of the patent: **04.07.2001**
(21) Application number: 92902905.6
(22) Date of filing: 17.01.1992
(51) Int. Cl.: C07K 14/78, A61K 38/39, A61L 15/32, A61L 31/00

(54) **MACROSCOPICALLY ORIENTED CELL ADHESION PROTEIN FOR WOUND TREATMENT**
MAKROSKOPISCH ORIENTIERTES ZELLADHÄSIONSPROTEIN ZUR WUNDBEHANDLUNG
PROTEINE D'ADHESION CELLULAIRE A ORIENTATION MACROSCOPIQUE POUR LE TRAITEMENT DES BLESSURES

(30) Priority: 18.01.1991 GB 9101191
(43) Date of publication of application: 03.11.1993
(73) Proprietor: Intercytex Limited, Manchester M13 9XX (GB)
(72) Inventor: BROWN, Robert, Dpt. of Experimental Pathology, Brockley Hill Stanmore Middlesex HA7 4LP (GB)
(74) Representative: Davies, Jonathan Mark
(86) International application number: GB9200100
(87) International publication number: WO9213003

(56) References cited:
- EP-A- 0 314 109
- US-A- 4 973 466
- Developmental Biology, vol. 88, no. 2, December 1981, (New York, US), M. CHIQUET et al.: "Muscle morphogenesis: Evidence for an organizing function of exogenous fibronectin", pages 220-235, see the whole article, especially page 230, column 2 - page 234

## Description

The present invention relates to materials for use in promoting wound healing, to processes for their production and their use in treating wounds in humans and animals.

There are four stages which can usually be identified in the natural healing process. Initially the wound is closed so as to limit blood loss and prevent infection. Then damaged tissue is removed and pathogens destroyed by phagocytosis. This is followed by granulation in which the wound is invaded by cell types appropriate to the surrounding tissue and scar formation occurs. Finally the scar tissue is remodelled and changes in the cell population occur resulting in a mature, healed wound. In any particular case variations from this general pattern will occur owing to factors such as the site and type of wound and the condition of the patient, and the details of the process, particularly the later stages are, as yet, not well understood.

Although very effective in most cases, the natural wound healing process can fail on occasion, or may be unsatisfactory, and medical intervention is desirable. Typical examples of failure include cases of severe burns involving substantial tissue damage where the wounds often do not even close completely and where skin grafts are required to secure granulation, cases of leg ulcers where, even when the wounds heal, the healed scar is physically weak and liable to break open very easily and cases where, although a wound would heal naturally, the scarring that remains may be unslightly or cause discomfort. Other wounds which frequently require intervention-are serious bone fractures and wounds to cartilage, ligaments and tendons which heal slowly or not at all or where the healed wound will not be sufficiently strong.

EP-A-0314109 describes a biodegradable collagen flake product and a biodegradable collagen sponge or sponge-like material which are useful for medical applications.

US 4973466 discloses wound healing dressings prepared by flocculating fibronectin, a biologically active fragment or an analogue thereof to produce a water-swellable gel. The gels, in the form of sheets, strips, wedges, strands or I-shaped cross-sections are especially useful in promoting the healing of corneal, scleral dermal, incisional wounds or lesions.

Despite considerable work over many years there have been no completely satisfactory treatments for many of these problems in wound healing.

The present inventors have developed macroscopically oriented materials, comprising a cell adhesion protein such as fibronectin, which, surprisingly have been shown to promote wound healing, in particular by creating a scaffolding to which the invading cells can adhere thus facilitating this stage of the wound healding procedure. Moreover, by aligning these materials with features of the wound or surrounding tissue, cell invasion may be directed along desired orientations thereby strengthening the initial repair and reducing the amount of reorientation required during the remodelling stage. Thus wound healing may be promoted and the mature healed wound can be made stronger or more cosmetically acceptable or both.

It is the orientation of the cell adhesion protein molecules on a macroscopic scale which is critical to the success of the materials of the invention in directing the wound healing process. In the past, investigations have been made using non-porous fibronectin obtained by precipitation from solution, however this results in at best very small scale orientation of fibronectin molecules and, usually, random orientation thereof, and such materials have no application in directing wound healing in accordance with the present invention.

The present invention therefore provides porous macroscopically oriented cell adhesion protein. Cell adhesion proteins useful in the present invention include fibronectin, vitronectin and von Willebrand protein (also called von Willebrand factor). Fibronectin is the preferred cell adhesion protein.

The invention further provides porous macroscopically oriented cell adhesion protein for use in methods of surgery or therapy practised on the human or animal body. The invention further provides the use of porous macroscopically oriented cell adhesion protein in the manufacture of medicaments, dressings or devices for use in methods of surgery or therapy practiced on the human or animal body. In particular aspects the methods of surgery or therapy involve promoting wound healing or directing wound healing or improving the appearance or strength of a healed wound or any combination of two or more thereof. The method of surgery or therapy may alternatively involve the growth of autograft material such as skin or ligament promoted or directed by porous macroscopically oriented cell adhesion protein.

The present invention also provides a method of treatment of a wounded human or animal comprising applying an effective non-toxic amount of porous macroscopically oriented cell adhesion protein to the wound.

The macroscopically oriented cell adhesion protein of the invention comprises large scale aggregates of cell adhesion protein, which self-assemble under favourable conditions as fibrils, the molecules in each individual fibril lying substantially parallel to each other, each individual fibril being oriented over a distance of at least 100 *µ*m and the fibrils being oriented substantially parallel to each other over macroscopic distances such as at least 0.1 mm, preferably 0.5 and most preferably for at least 1 mm. Individual fibrils may show orientation over a considerable distance, for instance up to 0.5 mm, possibly up to 1 mm or even for 5 mm or more, for instance 1,2,3 or 5 cm. The aggregate of fibrils may be oriented for over 5 mm or 1 cm or more, for instance 2,3, or 5 cm and, when prepared as a continuous web for subsequent division into individual dressings, the aggregate may be oriented over distances of many centimetres or even many metres.

In a simple embodiment of the invention the fibrils are oriented in a single direction and form a sheet or mat, possibly on a substrate for support, which may be applied to a wound. In more complex embodiments such sheets or mats may be laminated in non-parallel directions, for instance with the fibrils of one layer oriented at 90° to fibrils in a second layer. The fibrils may be arranged into fibres or may be formed on a substrate or oriented by fibres of a substrate, and such fibres may be formed into woven and nonwoven webs having at least one and often two or more orientation directions. When the oriented materials are formed by coating on a substrate, preferably the substrate will be a biodegradable or resorbable material such that it may be left in the wound and will eventually be destroyed as the wound heals or once it has healed or the substrate may be a physical support which is removed after formation of the oriented material.

In use the materials of the invention may be applied to wounds to direct and promote the cell invasion and thereby to increase the strength, cosmetic acceptability, healing time or other desirable characteristic of the healed wound. By way of example a simple unidirectionally oriented mat may be used with the orientation direction across the width of a linear wound in order to promote the closing of the wound and enhance the resistance to re-opening of the wound. In another example, more complex webs having multiple orientation directions may be used to promote regrowth of damaged tendons, intervertebral discs and corneas whilst directing adoption by the invading cells of orientations matched to that of the surrounding undamaged tissue or to recreate orientations of the original damaged tissue. Thus the use of the oriented materials of the invention will often involve aligning one or more orientation directions of the material with respect to features of the wound or surrounding tissues.

A particular application of the materials of the invention is in stimulation of new capillary growth, a frequently perceived objective for many forms of wound repair. Classically, the approach has been to attempt to stimulate angiogenesis generally, using a diffusible factor. However, one part of the process of angiogenesis is endothelial cell adhesion to and migration over the substrate matrix. A development of the present invention can be applied to this by promoting attachment/migration of capillary cells to discrete fibres or strands. These strands would be orientated in the direction of the required capillary growth. Strands can take the form of (i) pure fibronectin in macroscopic fibrous form; (ii) oriented Fn strands laid into conventional wound implant materials (e.g. gelatin or modified cellulose sponges); (iii) oriented fibronectin coated on braided resorbable sutures. Whether formed of oriented Fn or fibronectin coated, the individual strands should be less than 200 *µ*m wide (ideally between 1 and 100 *µ*m. These structures form excellent support and adhesion substrates for repair cells

In a further modification (particularly of the Fn-coated, braided suture) it is possible to incorporate a chemotactic stimulus by attaching a solid, growth factor containing gel to one end of the suture. A natural example of such a "gel" would be a blood or plasma clot (ideally prepared from the patient's own blood). Artificial substrates based on gelatin (or other gel-forming material) containing the required angiogenic factor could also be used. This suture would be drawn through or across the damaged tissue in such a way that new vessels would grow towards the end bearing the gel or clot. This form of suture may usefully be employed as an "angiogenic track" during repair of avascular or poorly vascular tissues such as torn menisci, ligaments or tendons.

Fibronectin for use in accordance with the invention may be obtained commercially in non-oriented form and may be oriented by processes such as are described below. Preferably substantially pure fibronectin is used. Other cell adhesion proteins are well known in the literature; again these are available in non-oriented form and require processing for instance as described below. The materials of the invention will usually be provided in sterile, pyrogen-free form.

Oriented materials according to the invention may further comprise additional therapeutic agents, for instance agents which promote wound healing such as growth factors and growth hormones, clotting factors, platelet adhesion promoters such as thrombin, agents which promote calcification, collagen, fibrinogen, antimicrobial agents and heparin.

The fibrils and oriented materials may be used as formed or stabilised by cross-linking using chemical reagents such as glutaraldehyde or enzymes such as factor XIIIa, which is a transglutaminase. Cross-linking with other components such as collagen and fibrinogen, for instance using a transglutaminase, is also contemplated. Where the materials of the invention include collagen and/or fibrinogen it is preferred for these also to be oriented substantially parallel to the fibrils of cell adhesion protein.

Preferably the oriented materials of the invention are used as, or as part of, a wound dressing, or are applied to open wounds separately from a conventional dressing. To derive improved strength and/or cosmetic acceptability of the mature wound it is preferred that the oriented materials are applied to the wound with the or an orientation direction aligned with features of the surrounding tissues so as to encourage invasion along the orientation direction. For instance, the fibres may be aligned with muscle fibres in the wound or underlying tissue, across a linear wound or parallel with or at right angles to directions in which a tissue will be strained once healed.

The invention further provides a process for producing porous macroscopically oriented cell adhesion protein materials which process comprises forming and orienting cell adhesion protein fibrils from solution and removing the solvent.

Solvents useful in accordance with the present process are generally aqueous solvents such as buffered water, distilled water, demineralised water and pyrogen-free water. The solvent may contain additional solutes and/or suspended particles for inclusion in or deposition on the fibronectin materials.

The solvent may be removed by evaporation, filtration concentration or by aggregating or precipitating the fibronectin using, for instance, appropriate concentrations of salts or by adjusting the pH of the solution to acidic or basic pH and collecting and drying the aggregate or precipitate. The oriented materials are preferably washed and dried and may be optionally stabilised, for instance by chemical cross-linking using reagents such as glutaraldehyde or enzymatically using factor XIIIa.

The cell adhesion protein may be oriented by self-association from solution, preferably a high concentration solution at 0.7mg/ml or greater, for instance greater than 1 mg/ml, such as at least 1.5 mg/ml, for instance 2 mg/ml or more or even up to 3 mg/ml or more at about neutral pH to form fibrils on solid surfaces which fibrils are sufficiently stable to be handled, recovered and dried. Use of a solution at about 1.5 mg/ml is most preferred. A pH of about 7.6, eg using tris-HCl buffer has been found convenient. Preferably the solution contains soluble ionic compounds to increase the ionic strength thereof, especially in the range up to 0.5 M ionic strength. Preferably the solution also contains urea at preferably 1 to 3 M. A combination of fibronectin, urea at 2 M and 0.1 to 0.5 M sodium chloride is preferred. Thus, for example, fibronectin may be oriented by applying continuous unidirectional motion, such as by stirring, to a saturated solution and removing the solvent so as to aggregate oriented fibronectin, for instance on the stirrer. This may be recovered and blotted to form mats which may be laminated in parallel or non-parallel directions to form a lattice. Alternatively, high concentration solutions may be drawn into fibres and the solvent removed leaving fibronectin fibres comprising oriented fibrils. A preferred technique for drawing fibres involves dipping an applicator onto the surface of the solution and lifting the applicator to produce one or more fibres under the effects of surface tension. A preferred material for the applicator is the mineral mica. In a further alternative, a concentrated solution of fibronectin is applied to a fibrous substrate and the solvent is removed.

Heparin can be incorporated into the fibronectin solution (preferably at ratios of 1:5 to 1:100, by weight heparin: Fn) without impairing its ability to form strands. However, after drying, strands made with higher heparin ratios (e.g. 1:5, heparin:Fn) were flat, with very little mass as a result of the high level of hydration of the newly formed strands due to the heparin content.

The invention will now be illustrated by the following Examples which are not intended to limit the scope of protection in any way.

### Example 1

A solution of human plasma fibronectin purified by gelatin affinity chromatography (approx 1.0 (eg 0.5 to 1.5) mg/ml) in neutral pH buffer (10 mM phosphate or 20 mM Tris HCl pH7.5) containing 0.15 M sodium chloride is placed into a pressurized "stirred cell" concentration device with an ultra filtration membrane (molecular weight cut off approx. 10 to 20,000 Daltons: eg Amicon PM 10 membrane). Such a stirred cell (eg 100 ml capacity) is operated at a preferred pressure of 172.4 kPa (range approx. 68.9 to 517.1 kPa) (25 psi (range approx. 10 to 75 psi)) under nitrogen or under air with a stirring rate of approx 300 rpm (range 50 to 600 rpm) at 4°C. The volume is slowly reduced under these conditions to less than half the initial volume giving a fibronectin concentration within the cell of approx 3 mg/ml (range of 2.0 to 10 mg/ml). The conditions for self-aggregation will vary depending on the purity and integrity of the fibronectin starting material, but within these ranges, a large clot or mat of solid fibronectin will be formed on the stirring bar of the cell. This can be removed and fresh fibronectin solution added to permit the formation of more fibronectin matting.

### Example 2

A starting solution as described in Example 1 but containing over 1mg/ml of fibronectin (Fn) at a pH around neutrality and sodium chloride concentration up to 0.2M is prepared. A suitable flat edged "applicator" (for example a 2cm glass cover slip) is dipped into the solution to a depth of at least 3mm. This same wetted edge is now touched onto a hydrophilic surface (e.g. flat plastic culture dish) forming a small pool of the Fn solution, clinging to both the "applicator" and the surface. When the applicator is slowly lifted off the surface to be coated, a single strand of protein forms between the "applicator" and "surface" under the effect of surface tension. This strand of protein (spanning between surface and applicator) can be pulled across the surface for 2 to 5mm and re-attached to the surface by again touching the applicator and the surface. The resultant strand of protein is firmly attached to the surface by multiple subdivided fibrils at either end. They are commonly 2 to 5 *µ*m in diameter and up to 5mm long. They are stable with or without chemical cross linking (e.g. with glutaraldehyde) and can be washed and dried without becoming dislodged. Their orientation on the "surface" can be controlled precisely.

In cell culture tests, strands of pure fibronectin promoted a directional orientation and attachment of fibroblasts in spite of the presence of soluble fibronectin. Stands of fibronectin were still visible in such cultures after 24 hrs exposure to fibroblasts.

### Example 3

Mats were prepared as described in Example 1 using a stirred cell, under a range of conditions to test for preferred composition of the starting fibronectin solution. Mat formation was assessed on the basis of the dry weight of mat recovered and uv absorbance (at 280nm) of the fibronectin solution at the start and end of the mat forming process. Fibronectin solutions made to 2M with urea were found to be preferable, giving a greater % mat formation at the same ionic strength.

The ionic strength of the Fn solution was raised in increments, using greater concentrations of sodium chloride from zero to 1.0 M, and the recovery of Fn as a mat (as % of total Fn in solution) was measured. From data on the relationship of sodium chloride concentration to % Fn incorporation to the mat, it is clear that mat formation is adequate between 0.1 M and 0.5 M sodium chloride with a preferred concentration of 0.1 M sodium chloride.

### Example 4

The influence of heparin, in the starting solution of Fn, was tested on the quantity and quality of mats formed in the "stirred cell" (see Example 1). As in Example 3, the efficiency of mat production was measured as % Fn incorporated into the aggregate. Heparin was added to known concentrations of Fn solution at ratios (weight:weight) from 1:15 to 1:200 (heparin:Fn). Heparin was from the Sigma Chemical Co., Poole, Dorset, U.K. Each mat was made under otherwise identical conditions, from solutions of Fn containing 0.1M sodium chloride, 2M urea, 50mM tris-HCl pH7.6. At ratios below 1:15 (Heparin:Fn) mat formation was largely or wholly inhibited. Beyond a ratio of 1:40 there was little change. The preferred ratio is 1:20 to 1:40. Heparin incorporation into the mat (measured by the "Methylene Blue" assay for glycosaminoglycans) was determined at approx. 20*µ* g/mg of Fn, using a starting solution Heparin:Fn ration of 1:15. This represents an incorporation rate of 30%. In general mats containing heparin had a poorer orientation than those prepared without. All of these materials, when dried, were convenient materials to place into wounds in a variety of tissues, rehydrating to form solid proteinaceous deposits in solutions at physiological ionic strength and pH.

## Claims

1. A porous macroscopically oriented cell adhesion protein, which protein is chosen from fibronectin, vitronectin and von Willebrand protein.

2. A protein according to claim 1 which is fibronectin.

3. A porous macroscopically oriented cell adhesion protein according to claim 1 or 2, for use in a method of surgery or therapy practised on the human or animal body.

4. Use of a porous macroscopically oriented cell adhesion protein according to claim 1 or 2, in the manufacture of a medicament, dressing or device for use in a method of surgery or therapy practised on the human or animal body.

5. Use according to claim 4 in a method of surgery or therapy comprising promoting wound healing or directing wound healing or improving the appearance or strength of a healed wound.

6. Use according to claim 4 involving the growth of autograft material promoted or directed by said protein.

7. A process for producing a porous macroscopically oriented cell adhesion protein according to claim 1 or 2, which process comprises forming and orienting cell adhesion protein fibrils from a solution thereof and removing the solvent.

8. A wound dressing comprising a porous macroscopically unidirectionally oriented cell adhesion protein according to claim 1 or 2.

9. A porous macroscopically oriented cell adhesion protein, which self-assembles as fibrils, the molecules in each individual fibril lying substantially parallel to each other, each individual fibril being oriented over a distance of at least 100 µm and the fibrils being oriented substantially parallel over macroscopic distances such as at least 0.1 mm, which protein is chosen from fibronectin, vitronectin and von Willebrand protein.

## Patentansprüche

1. Poröses, makroskopisch ausgerichtetes Zelladhäsionsprotein, das aus Fibronektin, Vitronektin und von Willebrand-Protein ausgewählt ist.

2. Protein nach Anspruch 1, das Fibronektin ist.

3. Poröses, makroskopisch ausgerichtetes Zelladhäsionsprotein nach Anspruch 1 oder 2 zur Verwendung in einem chirurgischen oder therapeutischen Verfahren, das am menschlichen oder tierischen Körper praktiziert wird.

4. Verwendung eines porösen, makroskopisch ausgerichteten Zelladhäsionsproteins nach Anspruch 1 oder 2 in der Herstellung eines Medikamentes, Verbandes oder einer Vorrichtung zur Verwendung in einem chirurgischen oder therapeutischen Verfahren, das am menschlichen oder tierischen Körper praktiziert wird.

5. Verwendung nach Anspruch 4 in einem chirurgischen oder therapeutischen Verfahren, umfassend das Fördern der Wundheilung oder Lenken der Wundheilung oder Verbessern des Aussehens oder der Stärke einer geheilten Wunde.

6. Verwendung nach Anspruch 4, umfassend das Wachstum von Autotransplantat, das von dem genannten Protein gefördert oder gelenkt wird.

7. Verfahren zum Herstellen eines porösen, makroskopisch ausgerichteten Zelladhäsionsproteins nach Anspruch 1 oder 2, wobei das Verfahren das Bilden und Ausrichten von Zelladhäsionsproteinfibrillen von einer Lösung davon und das Entfernen des Lösungsmittels umfasst.

8. Wundverband, umfassend ein poröses, makroskopisch und in einer Richtung ausgerichtetes Zelladhäsionsprotein nach Anspruch 1 oder 2.

9. Poröses, makroskopisch ausgerichtetes Zelladhäsionsprotein, das sich als Fibrillen selbst zusammensetzt, wobei die Moleküle in jeder einzelnen Fibrille im Wesentlichen parallel zueinander liegen, wobei jede einzelne Fibrille über eine Strecke von wenigstens 100 *µ*m ausgerichtet ist und die Fibrillen über makroskopische Strecken von wenigstens 0,1 mm im Wesentlichen parallel ausgerichtet sind, wobei das Protein aus Fibronektin, Vitronektin und von Willebrand-Protein ausgewählt ist.

## Revendications

1. Protéine poreuse d'adhérence des cellules macroscopiquement orientée, laquelle protéine est choisie parmi la fibronectine, la vitronectine et la protéine de von Willebrand.

2. Protéine selon la revendication 1 qui est de la fibronectine.

3. Protéine poreuse d'adhérence des cellules macroscopiquement orientée selon la revendication 1 ou 2, à utiliser dans une méthode de chirurgie ou de thérapie mise en oeuvre sur le corps humain ou animal.

4. Utilisation d'une protéine poreuse d'adhérence des cellules macroscopiquement orientée selon la revendication 1 ou 2, dans la fabrication d'un médicament, d'un pansement ou d'un dispositif à utiliser dans une méthode de chirurgie ou de thérapie mise en oeuvre sur le corps humain ou animal.

5. Utilisation selon la revendication 4 dans une méthode de chirurgie ou de thérapie comprenant favoriser la guérison des blessures ou diriger la guérison des blessures ou améliorer l'aspect ou la résistance d'une blessure guérie.

6. Utilisation selon la revendication 4 englobant la croissance d'une matière d'auto-greffe favorisée ou dirigée par ladite protéine.

7. Procédé pour produire une protéine poreuse d'adhérence des cellules macroscopiquement orientée selon la revendication 1 ou 2, lequel procédé comprend former et orienter des fibrilles protéiques d'adhérence des cellules à partir d'une solution de celles-ci et retirer le solvant.

8. Pansement pour blessure comprenant une protéine poreuse d'adhérence des cellules macroscopiquement orientée de manière unidirectionnelle selon la revendication 1 ou 2.

9. Protéine poreuse d'adhérence des cellules macroscopiquement orientée, qui s'assemble d'elle-même sous forme de fibrilles, les molécules de chaque fibrille individuelle reposant sensiblement parallèles les unes aux autres, chaque fibrille individuelle étant orientée sur une distance d'au moins 100 µm et les fibrilles étant orientées sensiblement parallèles sur des distances macroscopiques telles qu'au moins 0,1 mm, laquelle protéine est choisie parmi la fibronectine, la vitronectine et la protéine de von Willebrand.
